# EUROPEAN PATENT SPECIFICATION

(11) **EP 3 360 508 B1**
(45) Date of publication and mention of the grant of the patent: **30.10.2019**
(21) Application number: 17382070.5
(22) Date of filing: 13.02.2017
(51) Int. Cl.: A61C 17/06, A61C 5/90, A61C 19/00, A61G 13/10, A61B 1/24

(54) **AUXILIARY DEVICE FOR DENTAL PROCEDURE**
HILFSVORRICHTUNG FÜR ZAHNÄRZTLICHES VERFAHREN
DISPOSITIF AUXILIAIRE POUR PROCÉDURE DENTAIRE

(43) Date of publication of application: 15.08.2018
(73) Proprietor: Astradentium Health Technologies, S.L., 08003 Barcelona (ES)
(72) Inventor: LLORO BOADA, Victor Alejandro, 08860 Castelldefels (ES); LLORO BOADA, Ivan, 08860 Castelldefels (ES)
(74) Representative: Gallego Jiménez, José Fernando

(56) References cited:
- JP-A- H1 028 696
- JP-A- 2004 033 474
- US-A- 3 537 447
- US-A- 3 735 491
- US-A- 4 967 320
- US-A- 5 127 411
- US-A1- 2005 227 199

## Description

### Technical field

The present invention generally relates to the field of dental devices. In particular, the invention relates to an auxiliary device for dental procedure that does not obstruct a patient's buccal orifice, thereby enabling a healthcare professional to work normally during the dental procedure, being able to treat any accessible space or tissue through the mouth of the patient.

### Background of the Invention

US-B2-7300401 discloses a device for dental use that is placed in the mouth of a patient during the dental procedure for retraction of the mouth and expulsion of saliva. Said device includes a frontal suction part, a right cheek retraction part, a left cheek retraction portion, and an aspiration portion. Furthermore, the device consists of a rigid single piece of elastic and tubular material, having a cylindrical shape or another cross-sectional shape.

US-B2-7785105 discloses a device for keeping the operating field dry during a dental procedure. The device includes a U-shaped member for securing the patient's tongue during the dental procedure. In addition, it includes lip retractors that extend in an arched disposition around the cheeks. In some embodiments the device also includes a saliva ejector.

According to Chinese utility model CN-Y-201115660, a device with lateral retractors for opening the mouth of a patient during a dental procedure is known, such device including a saliva aspiration function by means of two tubular mechanisms for filtration /evacuation of the saliva, which rest positioned in the back of the patient's mouth during the procedure. With the aforementioned aspiration function contamination can be reduced due to the air present in the room or due to an inhaler.

EP-B1-1131015 also discloses an intraoral lighting dental device which enables the interior of the buccal cavity of a patient to be lighted during a dental procedure and thereby avoiding lighting issues. The lighting device comprises an occlusion apparatus designed to fit into the patient's dentition so that during the dental procedure the patient can rest his jaws and the device is fixed in his mouth. Moreover, the dental device has a retractor for the tongue and cheeks that allows isolation and protection of the tongue and tissue from the patient's cheeks during the medical procedure. The device may also include fluid extraction channels for extracting the fluids present in the interior of the patient's buccal cavity during the dental procedure.

US-A-3396468 discloses a device applicable in the buccal cavity for dental procedures, said device eliminates the fluids and can be easily and quickly inserted into the patient's mouth.

US-A-4053984 discloses a removable dental device, which allows the patient's mouth to be opened for dental work while maintaining a dry and wide operation field.

US-A-4967320 discloses an air emitting light and shielding apparatus for dental treatment. The apparatus is comprised of a pressurized air source connected to an adjustably positionable air emitting light formed so as to develop an air envelope substantially surrounding the face and oral cavity or other body parts of a patient residing in a selected position in the dental chair or other operating table including positions in which the patient may be tilted such that vapors, blood, saliva splash, dental particles, or other foreign matter propelled from the patient's oral cavity during drilling, air-water syringing, or the like, are deflected by the inner boundary of the air envelope and are transported downstream of the patient's oral cavity for collection on a disposable patient drape thereby preventing the same from reaching the face and respiratory tract of a dental team member operating on the patient.

US3537447, JP2004033474 and JPH1028696 also disclose devices for dental procedures forming a protective shield between a dentist and a patient.

US-A-5127411 discloses an apparatus to aid in isolating a medical practitioner from infectious materials found in a patient. The apparatus includes a collector for providing a vacuum barrier around the mouth of a patient to trap aerosols and the like emanating from the patient during a dental procedure. The collector is flow connected to a vacuum source for drawing a vacuum, and gases drawn in by the source are passed through a filter. The collector is ring-shaped to surround the mouth and to allow the practitioner to see into the site of the procedure and is disposable after each operation.

However, there is at present no known device for dental procedure which is held inside or in the mouth of a patient during the dental procedure and which includes a barrier mechanism between the inside and the outside of the patient's mouth formed by one or more ducts including an opening at one of its ends which, by generating a gaseous fluid flow or air curtain, provides greater protection by allowing the filtration of pathogens during the dental procedure.

### Disclosure of the invention

The present invention provides an auxiliary device for dental procedure as defined in claim 1. Preferred embodiments are defined in the dependent claims 1. The auxiliary device is intended to be applied to or adjacent to a patient's perioral area, i.e. the space comprised by the volume of 3 cm around the entire length of the lip vermilion, including commissure. The proposed auxiliary device does not obstruct the buccal orifice so that a healthcare professional can work normally by treating any space or tissue accessible through the patient's mouth (such as the oral cavity, nasal cavity, larynx, pharynx, esophagus or maxillary sinus).

The proposed auxiliary device characteristically comprises a gaseous fluid flow-generating member, or air curtain, which exerts a barrier effect between an interior area of the patient's mouth and the outside of the mouth, and a support of said gaseous flow generating member which maintains it placed in an area proximate to said perioral area. Said generating member is formed by at least one duct including at one of its ends an opening which is placed in the exterior of the perioral area, in front of said support. Herein, the proposed auxiliary device prevents or minimizes the passage of sprays, micro-droplets, drops, or other particles, as well as pathogens (such as viruses, fungi, bacteria, prions, etc.) from the interior of the buccal cavity to the exterior, and vice versa, thus avoiding direct cross-contamination between the patient and the healthcare professional, and vice versa, as well as indirect contamination (that is to say, through the environmental contamination of a dental clinic or work area of a healthcare professional or tooling, etc. in such a way that the infection of the patient or healthcare professional can originate due to a previous patient). Likewise, the proposed auxiliary device allows the healthcare professional to work normally and unobstructed throughout the entire buccal cavity, and can be used autonomously or in conjunction with a rubber dam.

The proposed auxiliary device also prevents particles resulting from the removal of potentially hazardous dental restorative materials (for example, dental amalgams) from leaving the patient's mouth, which up to date is solved using elaborate methods based on a combination of suction hoods, rubber dams and specialized masks.

Preferably, the gaseous flow is a laminar flow.

Preferably, said aperture has an elongate shape with a central portion of greater width. Further, the generating member may be attached to the holder in a central portion, or, alternatively, in a lateral portion.

In one exemplary embodiment, another end of said duct is connectable to an aspiration or pressure-blowing pump of a gaseous fluid selected from air or other neutral gas.

Optionally, the aspiration or pressure-blowing pump may also be associated with a circuit in which an anti-pathogen agent will be provided.

In one exemplary embodiment said generating member comprises two ducts, each including at one of its ends said aperture, and being connected at the other end thereof to a single tubular section which is connectable to an aspiration or pressure- blowing pump of a gaseous fluid selected from air or other neutral gas. According to this embodiment, optionally, the aspiration or pressure-blowing pump may also be associated with a circuit in which an anti-pathogen agent will be provided.

In one exemplary embodiment, the support further includes one or more supports for cannulas or saliva aspiration ejectors.

In one exemplary embodiment, the cited support includes a buccal expansion element comprising at least a portion providing props in distal areas of the mouth for cheek separation (buccal mucosa expansion) during the dental procedure. Alternatively, in another embodiment, the support includes a buccal expansion member comprising at least a portion which provides lip-shaped props for lip retraction during the dental procedure.

Said mouth expansion element may be formed of two identical, arcuate portions or parts which engage in distal, lateral, or alternatively, labial or lateral areas of the patient's mouth.

Moreover, the aspiration or pressure-blowing pump may be associated with flow control means.

In still yet another embodiment, the device includes or has a support part for a lighting unit, for example a LED or a light wave guide such as an optical fiber, to light up the interior of the oral cavity of the mouth of the patient during the dental procedure.

The proposed auxiliary device can be used for protection between the patient and a healthcare professional, and vice versa, due to cross-contamination of biological type by direct contagion including Flügge micro-droplets or micro-droplets produced by a cooling spray, or indirect via contamination of surfaces, wardrobe or instrumental of a dental box.

Likewise, the auxiliary device may also be used for protection between the patient and the healthcare professional, and vice versa, due to cross-contamination of the chemical type through direct contact or intoxication including suspended particles of dental powder or dental reconstruction material such as resins, amalgams, composites or gases produced by gutta-percha incineration, or indirect contamination of surfaces, clothing or instruments of a dental box.

The auxiliary device may also be used to prevent water soaking or wetting of a dental instrument into the patient's oral cavity including materials, surfaces, tissues and instruments, or even to protect the healthcare professional from the patient's halitosis.

### Brief description of the drawings

The above and other features and advantages of the present invention will become more apparent from the following detailed description of exemplary embodiments, which are merely illustrative and not limitative, with reference to the accompanying drawings, in which:
Figs. 1 and 2 show different views of the proposed auxiliary device for dental procedure according to a preferred embodiment of the present invention;
Fig. 3 shows an alternative example of an auxiliary device for dental procedures, in which the support includes supports for saliva aspirating cannulas or ejectors.

### Detailed description of exemplary embodiments

The present invention will be described in more detail by reference to the following embodiments which are presented for purpose of illustration and should not be constructed to limit the scope of the invention thereto.

The present invention provides an auxiliary device for dental procedures, referenced as 1 in the figures, which allows the filtering of pathogens or other particles by generation, when the auxiliary device is positioned inside the mouth of a patient during said dental procedure, of a gaseous fluid flow, for example laminar, thereby exerting a barrier effect between the inside and the outside of the patient's mouth.

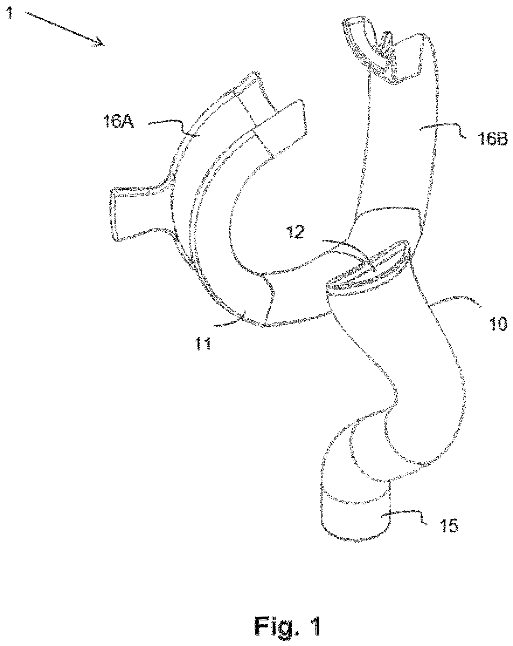

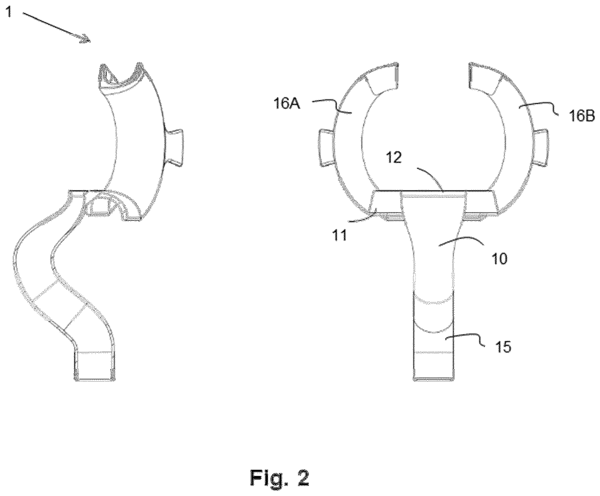

Figs. 1 and 2 show a first exemplary embodiment of the proposed auxiliary device 1 constructed in accordance with the present invention. As can be seen, the auxiliary device 1 includes a generating member 10 of said gaseous fluid flow and a support 11 for holding the generating member 10 placed in an area proximate to the perioral area. According to this first embodiment, the generating member 10 is attached to the support 11 in a central portion, although this is not limiting since, in other examples, in this case not shown, the generating member 10 may be attached to the support 11 in a lateral portion. Also according to this exemplary embodiment, the generating member 10 is formed by a duct including at one of its ends an opening 12 which is placed in the exterior of the perioral area in front of said support. The aperture 12 may be of different dimensions, but preferably, it is elongated with a central portion of greater width as shown in the figures.

The support 11 comprises a buccal expansion element, formed by two identical or arcuate portions or parts 16A, 16B, which provide supports in distal areas of the patient's mouth to allow separation of the cheeks and lips (lip retraction) during the dental procedure. Also, the auxiliary device 1 includes a tubular section 15 connectable to one or more aspiration or blow-off air intakes available in a dental chair (not illustrated) or, alternatively, to an aspiration or pressure-blowing pump of a gaseous fluid (not illustrated), for example air or, alternatively, another neutral gas.

Said aspiration or pressure-blowing pump of a gaseous fluid is preferably associated with flow control means. Optionally, the aspiration or pressure-blowing pump may also be associated with a circuit (not illustrated) which provides an anti-pathogenic agent.

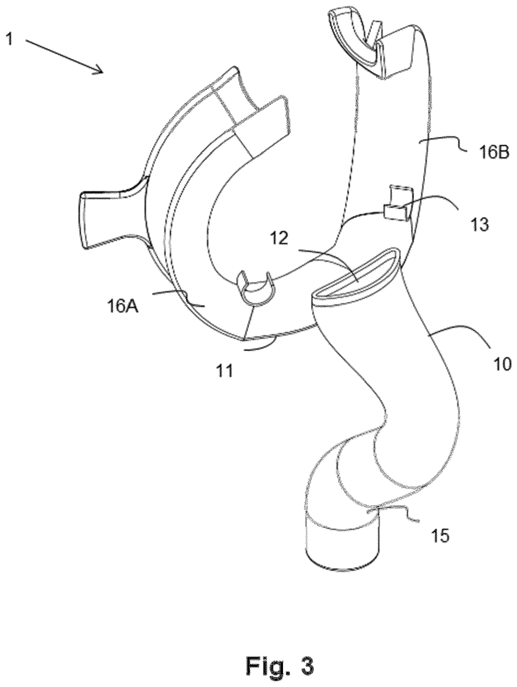

Referring to Fig. 3, a second exemplary embodiment of the proposed auxiliary device 1 is shown. The auxiliary device 1 of this exemplary embodiment is the same as described above with the particularity that the support 11 includes two supports or props 13 for supporting saliva aspiration cannulas or ejectors. Alternatively, instead of including two props 13, a single support 13 could be included.

According to another exemplary embodiment, in this case not illustrated, the generating member 10 comprises two ducts, each one including at one of its ends said aperture 12, and being connected by the other of its ends to the tubular section 15, by forming a 'Y'.

Said aspiration or pressure-blowing pump of a gaseous fluid is preferably associated with flow control means, and a circuit for supplying said anti-pathogen chemical.

In still other alternative embodiments of the proposed auxiliary device 1, also not illustrated by the simplicity of the figures, further includes a lighting unit to light up the interior of the oral cavity of the patient's mouth during the dental procedure. In one exemplary embodiment, said lighting unit comprises lighting elements for example of LED type (light-emitting diode) and is supplied by a source of electrical energy (such as batteries or a generator) included in the auxiliary device itself or externally thereto. Alternatively, said lighting unit comprises a light waveguide such as an optical fiber.

The proposed auxiliary device 1 can come in different sizes and accommodate different shapes and sizes of the mouth.

The scope of the present invention is defined in the appended claims.

## Claims

1. Auxiliary device for dental procedure, said device (1) comprising a support (11) intended to be placed in a perioral area of a patient, **characterized in that** the device further comprises a generating member (10) of a gaseous fluid flow exerting a barrier effect between an inner area of the patient's mouth and the outside of the mouth, said generating member (10) of a gaseous fluid flow being formed by at least one duct including at one of its ends an aperture (12) that is placed in the exterior of the perioral area of the patient in front of said support (11), and the generating member (10) being attached to the support (11) in a central portion.

2. The device according to claim 1, wherein said aperture (12) is elongated and has a central portion of greater width.

3. The device according to claims 1 or 2, wherein another end of said duct is connectable to an aspiration or pressure-blowing pump of a gaseous fluid selected from air or other neutral gas.

4. The device according to claims 1 or 2, wherein said generating member (10) comprises two ducts, each one including at one of its ends said aperture (12), and being connected at the other end to a single tubular section (15) which is connectable to an aspiration or pressure-blowing pump of a gaseous fluid selected from air or other neutral gas.

5. The device of any one of the preceding claims, wherein said support (11) includes a buccal expansion element comprising at least a portion which provides props in distal areas of the patient's mouth for separation of the cheeks during said dental procedure.

6. The device of any of the preceding claims 1 to 4, wherein said support (11) includes a buccal expansion element comprising at least a portion which provides props in labial areas of the patient's mouth for lip retraction during said dental procedure.

7. The device of claim 1, wherein the gaseous fluid flow is a laminar flow.

8. The device of claims 5 or 6, wherein said support (11) further includes at least one support (13) for saliva aspiration cannulas or ejectors.

9. The device of claim 5, wherein the buccal expansion element is formed by two identical, arcuate portions or pieces which engage in distal, lateral areas of the patient's mouth.

10. The device of claim 6, wherein the buccal expansion element is formed by two identical, arcuate portions or pieces which engage in labial, lateral areas of the patient's mouth.

11. The device of claims 3 or 4, wherein said aspiration or pressure-blowing pump is associated with flow control means.

12. The device of claims 3, 4 or 11, wherein said aspiration or pressure-blowing pump is associated with a circuit providing an anti-pathogenic agent.

13. The device of any one of the preceding claims, further comprising a lighting unit to light up the interior of the oral cavity of the patient's mouth during the dental procedure.

14. The device of claim 13, wherein said lighting unit comprises LED-type lighting elements or a light-waveguide.

## Patentansprüche

1. Hilfsvorrichtung für zahnärztliche Eingriffe, wobei die Vorrichtung (1) eine Stütze (11) umfasst, die dazu bestimmt ist, in einem perioralen Bereich eines Patienten platziert zu werden, **dadurch gekennzeichnet, dass** die Vorrichtung ferner ein Erzeugungselement (10) eines gasförmigen Fluidstroms umfasst, das eine Barrierewirkung zwischen einem inneren Bereich des Mundes des Patienten und der Außenseite des Mundes ausübt, wobei das Erzeugungselement (10) eines gasförmigen Fluidstroms durch mindestens einen Kanal gebildet wird, der an einem seiner Enden eine Öffnung (12) aufweist, die außerhalb des perioralen Bereichs des Patienten vor der Stütze (11) platziert ist, und wobei das Erzeugungselement (10) an dem Träger (11) in einem zentralen Abschnitt angebracht ist.

2. Vorrichtung nach Anspruch 1, wobei die Öffnung (12) länglich ist und einen zentralen Abschnitt größerer Breite aufweist.

3. Vorrichtung nach Anspruch 1 oder 2, wobei ein anderes Ende des Kanals mit einer Ansaug- oder Druckblaspumpe eines gasförmigen Fluids verbunden werden kann, das aus Luft oder einem anderen neutralen Gas ausgewählt ist.

4. Vorrichtung nach Anspruch 1 oder 2, wobei das Erzeugungselement (10) zwei Kanäle aufweist, von denen jeder an einem seiner Enden die Öffnung (12) aufweist und am anderen Ende mit einem einzelnen rohrförmigen Abschnitt (15) verbunden ist, der mit einer Ansaug- oder Druckblaspumpe eines gasförmigen Fluids verbunden werden kann, das aus Luft oder einem anderen neutralen Gas ausgewählt ist.

5. Vorrichtung nach einem der vorhergehenden Ansprüche, wobei die Stütze (11) ein bukkales Expansionselement aufweist, das mindestens einen Abschnitt umfasst, der in distalen Bereichen des Patientenmundes Abstützungen zum Trennen der Wangen während des zahnärztlichen Eingriffs bereitstellt.

6. Vorrichtung nach einem der Ansprüche 1 bis 4, wobei die Stütze (11) ein bukkales Expansionselement umfasst, das mindestens einen Abschnitt umfasst, der in labialen Bereichen des Patientenmundes Abstützungen zum Zurückziehen der Lippen während des zahnärztlichen Eingriffs bereitstellt.

7. Vorrichtung nach Anspruch 1, wobei das gasförmige Fluid ein Laminarfluss ist.

8. Vorrichtung nach einem der Ansprüche 5 oder 6, wobei die Stütze (11) ferner mindestens eine Stütze (13) für Speichelabsaugskanülen oder -ejektoren aufweist.

9. Vorrichtung nach Anspruch 5, wobei das bukkale Expansionselement durch zwei identische bogenförmige Abschnitte oder Stücke gebildet ist, die in distale laterale Bereiche des Patientenmundes eingreifen.

10. Vorrichtung nach Anspruch 6, wobei das bukkale Expansionselement durch zwei identische bogenförmige Abschnitte oder Stücke gebildet ist, die laterale innere Lippenbereiche des Patientenmundes eingreifen.

11. Vorrichtung nach Anspruch 3 oder 4, wobei die Ansaug- oder Druckblaspumpe mit Flusssteuermitteln verknüpft ist.

12. Vorrichtung nach Anspruch 3, 4 oder 11, wobei die Ansaug- oder Druckblaspumpe mit einer Schaltung verknüpft ist, die einen antipathogenen Wirkstoff bereitstellt.

13. Vorrichtung nach einem der vorhergehenden Ansprüche, ferner umfassend eine Beleuchtungseinheit zum Beleuchten des Innenraums der Mundhöhle des Patienten während des zahnärztlichen Eingriffs.

14. Vorrichtung nach Anspruch 13, wobei die Beleuchtungseinheit LEDartige Beleuchtungselemente oder einen Lichtwellenleiter umfasst.

## Revendications

1. Dispositif auxiliaire pour procédure dentaire, ledit dispositif (1) comprenant un support (11) destiné à être placé dans une zone péri-orale d'un patient, **caractérisé en ce que** le dispositif comprend en outre un élément générateur (10) d'un flux de fluide gazeux exerçant un effet de barrière entre une zone interne de la bouche du patient et l'extérieur de la bouche, ledit élément générateur (10) d'un flux de fluide gazeux étant constitué d'au moins un conduit comportant à l'une de ses extrémités une ouverture (12) qui est placée à l'extérieur de la zone péri-orale du patient en face dudit support (11), et l'élément générateur (10) étant fixé au support (11) dans une partie centrale.

2. Dispositif selon la revendication 1, dans lequel ladite ouverture (12) est allongée et a une partie centrale de plus grande largeur.

3. Dispositif selon les revendications 1 ou 2, dans lequel une autre extrémité dudit conduit peut être raccordée à une pompe d'aspiration ou de soufflage sous pression d'un fluide gazeux choisi parmi l'air ou un autre gaz neutre.

4. Dispositif selon les revendications 1 ou 2, dans lequel ledit élément générateur (10) comprend deux conduits, chacun comportant à l'une de ses extrémités ladite ouverture (12), et étant raccordé à l'autre extrémité à une section tubulaire unique (15) qui peut être raccordée à une pompe d'aspiration ou de soufflage sous pression d'un fluide gazeux choisi parmi l'air ou un autre neutre gaz.

5. Dispositif de l'une quelconque des revendications précédentes, dans lequel ledit support (11) comporte un élément d'expansion buccale comprenant au moins une partie qui fournit des cales dans les zones distales de la bouche du patient pour la séparation des joues pendant ladite procédure dentaire.

6. Dispositif de l'une quelconque des revendications précédentes 1 à 4, dans lequel ledit support (11) comporte un élément d'expansion buccale comprenant au moins une partie qui fournit des cales dans les zones labiales de la bouche du patient pour la rétraction de la lèvre pendant ladite procédure dentaire.

7. Dispositif de la revendication 1, dans lequel le flux de fluide gazeux est un flux laminaire.

8. Dispositif des revendications 5 ou 6, dans lequel ledit support (11) comporte en outre au moins un support (13) pour des canules d'aspiration ou extracteurs de salive.

9. Dispositif de la revendication 5, dans lequel l'élément d'expansion buccale est constitué de deux parties ou pièces identiques, arquées qui sont introduites dans les zones distales, latérales de la bouche du patient.

10. Dispositif de la revendication 6, dans lequel l'élément d'expansion buccale est constitué de deux parties ou pièces identiques, arquées qui sont introduites dans les zones labiales, latérales de la bouche du patient.

11. Dispositif des revendications 3 ou 4, dans lequel ladite pompe d'aspiration ou de soufflage sous pression est associée à des moyens de régulation de flux.

12. Dispositif des revendications 3, 4 ou 11, dans lequel ladite pompe d'aspiration ou de soufflage sous pression est associée à un circuit fournissant un agent anti-pathogène.

13. Dispositif de l'une quelconque des revendications précédentes, comprenant en outre une unité d'éclairage pour éclairer l'intérieur de la cavité buccale de la bouche du patient pendant la procédure dentaire.

14. Dispositif de la revendication 13, dans lequel ladite unité d'éclairage comprend des éléments d'éclairage du type à LED ou un guide d'ondes lumineuses.
